# EUROPEAN PATENT SPECIFICATION

(11) **EP 2 621 889 B1**
(45) Date of publication and mention of the grant of the patent: **17.07.2019**
(21) Application number: 10760618.8
(22) Date of filing: 01.10.2010
(51) Int. Cl.: C07C 215/20

(54) **PROCESS FOR MAKING FINGOLIMOD HYDROCHLORIDE CRYSTALS**
VERFAHREN ZUR HERSTELLUNG VON FINGOLIMOD-HYDROCHLORIDKRISTALLEN
PROCÉDÉ DE FABRICATION DE CRISTAUX DE CHLORHYDRATE DE FINGOLIMOD

(43) Date of publication of application: 07.08.2013
(73) Proprietor: Synthon BV, 6545 CM Nijmegen (NL)
(72) Inventor: WESTHEIM, Raymond Jozef Hubertus, 5345 JT Oss (NL)
(74) Representative: Mendivil Gil, Maria Dolores
(86) International application number: PCT/EP2010/006038
(87) International publication number: WO 2012/041358

(56) References cited:
- WO-A1-00/27798
- WO-A2-2010/055028
- CN-A- 1 528 738
- CN-A- 1 814 583
- JP-A- H11 310 556
- US-A- 5 604 229
- US-A1- 2001 008 945
- US-A1- 2003 229 251
- DATABASE WPI Week 200501 Thomson Scientific, London, GB; AN 2005-000435 XP002634962, & CN 1 528 738 A (MA Q) 15 September 2004 (2004-09-15)
- GILENYA ET AL: US HIGHLIGHTS OF PRESCRIBING INFORMATION, 21 September 2010 (2010-09-21), pages 1-16,

## Description

Fingolimod (often coded as FTY 720), chemically 2-amino-2-[2-(4-octylphenyl)ethyl]-propane-1,3-diol of the formula (1) 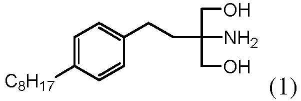 is a pharmaceutically active compound currently tested as an immunosuppressive drug and as an active agent in treatment of multiple sclerosis. It may form stable acid addition salts, of which fingolimod hydrochloride is the most common one.

Fingolimod has been first disclosed in EP 627 406 of Yoshitomi , where also two basic routes for making it have been described.

Fingolimod hydrochloride exists in a stable crystalline form. Such crystalline product was first obtained in the above EP '406 by recrystallization of fingolimod hydrochloride from a solution thereof in ethanol. In Example 5 of WO 2000/027798, crystals of fingolimod hydrochloride were obtained by crystallization from a mixture of ethyl acetate and ethanol. In Example 3 thereof, crystals were obtained by precipitation after concentration of the ethanolic solution. JP-H-11310556 discloses the use of ethanol and ethyl ether for recrystallizing fingolimod hydrochloride.

It was found by the present inventor after repeating the crystallization processes of the prior art that fingolimod hydrochloride is obtainable only as a waxy- or a cotton-like solid with poor filterability and flowability. Because of these properties, such crystal habit is inconvenient for large scale processing and for subsequent formulation into pharmaceutical compositions.

Thus, while processes for making crystalline fingolimod hydrochloride are known in the art, an improvement in the matter is still desirable.

### Brief description of the present invention

The present invention is based on a discovery of an improved process for making crystalline fingolimod hydrochloride.

In a first aspect, the present invention provides for a process of making crystalline fingolimod hydrochloride of formula (1a), 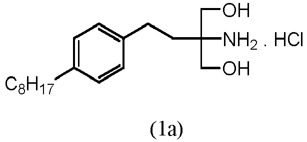 comprising a step of contacting a solution of fingolimod hydrochloride in a solvent with an antisolvent, wherein the antisolvent is a C5-C10 aliphatic, cyclic or aromatic hydrocarbon, or C4-C10 aliphatic ether, whereby crystalline fingolimod hydrochloride precipitates, and isolating crystalline fingolimod hydrochloride from the formed liquid medium..

In a preferred aspect, the temperature of the solution of fingolimod hydrochloride in the solvent is from 15 °C to the reflux temperature and/or the temperature of the contact with the antisolvent is from 0°C to 40 °C.

### Brief description of drawings

Fig. 1 XRPD pattern of "Form I" fingolimod hydrochloride
Fig. 2 XRPD pattern of "Form II" fingolimod hydrochloride
Fig. 3 XRPD pattern of "Form III" fingolimod hydrochloride

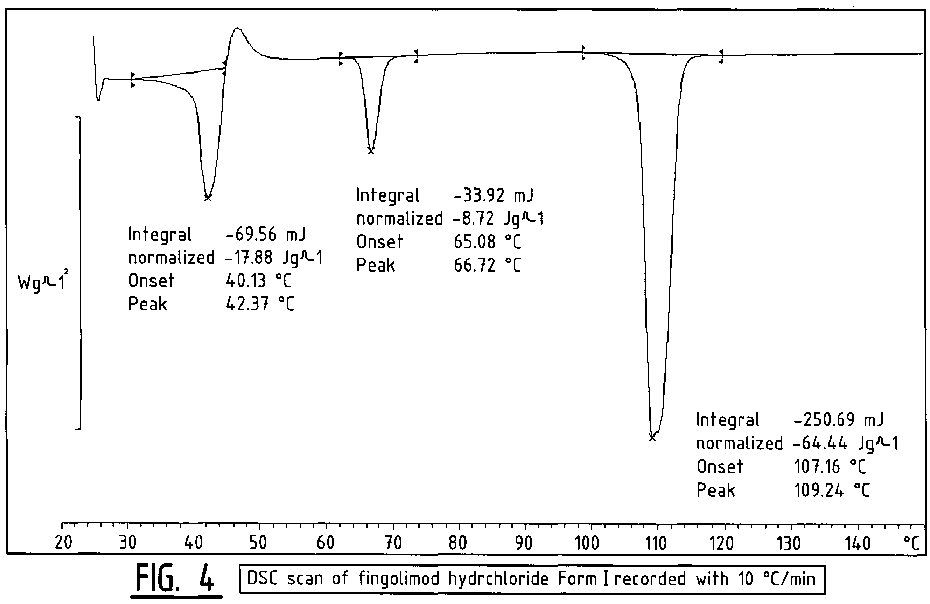

Fig.4 DSC csan of "Form I" finglolimod hydrochloride

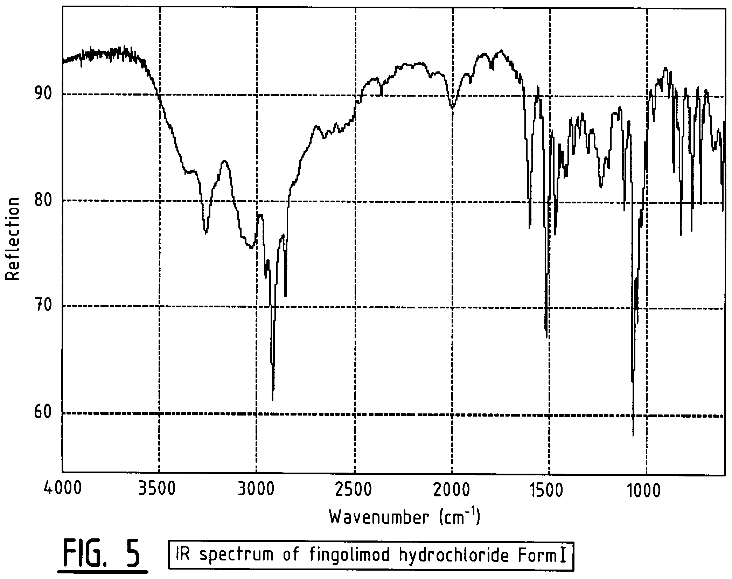

Fig.5 IR spectrum of :Form I" fingolimod hydrochloride

### Detailed description of the present invention

The crystalline fingolimod hydrochloride obtainable by processes of the prior art documents disclosed above is characterized by a distinctive XRPD pattern and IR spectrum, which allow to conclude that each of these processes provides a stable crystalline form, which is denoted for purpose of the present invention as Form I.

The process of our invention provides the same crystalline form, however in a better habit which results in better filterability and flowability. In particular, the population of crystals obtainable by the process of the present invention is characterised by small regular crystals with low tendency to adhere together and to adsorb solvent on the surface of the crystals.

The general feature characterizing the process of the present invention is in that the crystalline fingolimod hydrochloride does not crystallize from an oversaturated solution in a solvent under a relatively slow cooling rate, thus inadvertently causing stepwise growth of crystals with undesirable properties. Instead, crystals of fingolimod hydrochloride are obtained by a rapid precipitation in a liquid medium, in which the compound is inherently insoluble, whereby large amount of small crystals with no possibility of their further growth are obtained. Such crystals are more regular in shape and thus they exhibit good filterability and flowability after drying. As a result, isolation and elaboration procedures of the solid fingolimod hydrochloride are shorter, which is advantageous, in particular , at a large scale production process.

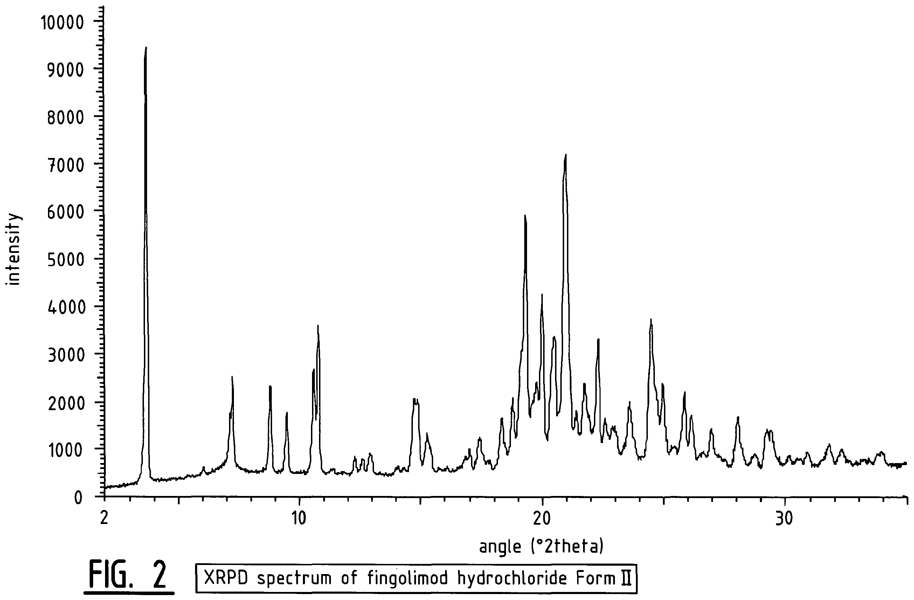

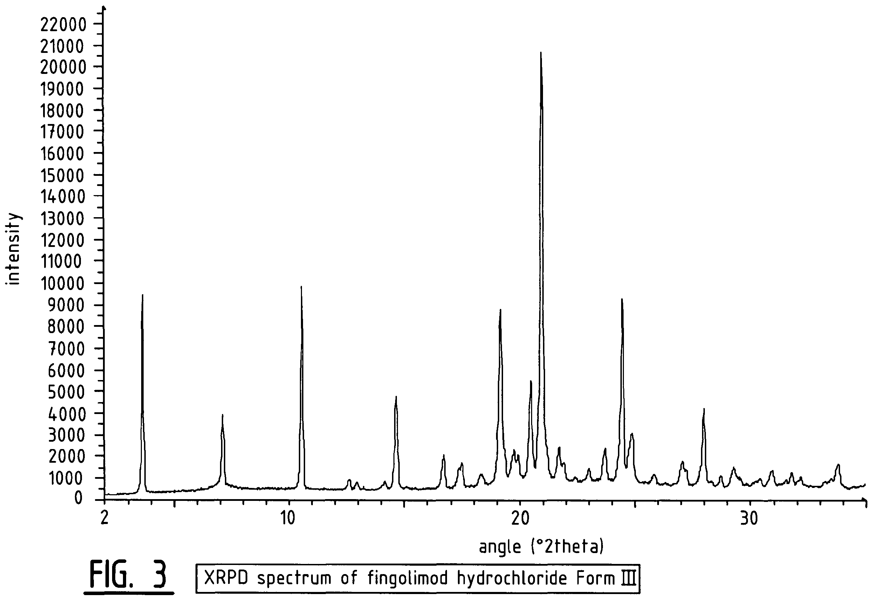

The second advantageous feature of the process of the present invention is in that it provides the crystalline Form I defined above with no contamination with other crystalline forms. Careful study of solid state properties of the crystalline fingolimod hydrochloride performed by the present inventor has confirmed the teaching recently disclosed in WO 2010/055028 shown that fingolimod hydrochloride may form metastable crystalline forms upon heating. At least two crystalline forms , hereby denoted as Form II and Form III, respectively (see Figures 2 and 3), are formed after heating the Form I at 40-60 °C or 80 °C, resp. Heating at more than 107°C provides a solid-liquid transition resulting in a liquid crystal form, hereby denoted as T1. Each of the metastable form converts to the stable Form I after cooling, however, at least in case of the Form II, such conversion is very slow. Thus, precipitating fingolimod hydrochloride at temperatures higher than 40°C, which is the typical feature of the prior art crystallization procedures, may produce fingolimod hydrochloride as a mixture of crystalline forms, which is undesirable. The second production possibility known in the art, namely crystallization from a solution that has been concentrated by evaporation, has yet another disadvantage that solvated crystals and/or liquid crystals might be formed in case of too high degree of concentration (such forms have been detected after thorough concentration of solutions of fingolimod hydrochloride in water or in methanol) . Such lyotropic crystal forms are metastable, they however might impurify the desired Form I (and thus alter the physical properties thereof) in case of incomplete removal of the solvent.

In the first technological step of the process of the present invention, a solution of fingolimod hydrochloride in a solvent is provided. The starting fingolimod hydrochloride may preferably be an isolated solid material obtained after separation of the solid from a reaction mixture comprising it. Alternately, a reaction mixture comprising fingolimod hydrochloride and the solvent (as defined hereinafter) may be used as well.

The "solvent" as used throughout the present invention, is a liquid, in which fingolimod hydrochloride is sufficiently soluble, at least at enhanced temperature. Preferably, the concentration of fingolimod hydrochloride in the solvent at such temperature is higher than 2.5 g/ 100 ml, advantageously higher than 10g/ 100 ml.

The solvent of the present invention is preferably an aliphatic alcohol of 1 to 6 carbon atoms, such as methanol, ethanol, n-propanol, isopropanol, and/or n-butanol, and mixtures thereof with water .

In the second technological step, the solution of fingolimod hydrochloride in the solvent, which is kept at the temperature within the range from 15 °C and the reflux temperature, is contacted with an antisolvent under stirring.

The "antisolvent" is an organic liquid, in which fingolimod hydrochloride is essentially insoluble. Furthermore, the antisolvent should be preferably fully miscible with the solvent. Typical antisolvent is a C5-C10 aliphatic, cyclic or aromatic hydrocarbon, e.g. hexane, heptane, cyclohexane, benzene, toluene etc., or C4-C10 aliphatic ether, e.g. diethyl ether or di-isopropyl ether. It is preferred that the solution of fingolimod hydrochloride in the solvent is slowly poured into stirred antisolvent, not vice versa. The temperature of the antisolvent is preferably less than 40°C, advantageously in the range from 0 to 40°C. Useful ratio between the solvent and antisolvent is from 1:2 to 1: 20 (v/v), typically 1 : 4 to 1: 10 (v/v). The antisolvent may be advantageously seeded with seed crystals of the Form I of fingolimod hydrochloride. In an advantageous mode, the formed suspension in the solvent/antisolvent mixture is stirred after completing the mixing process for some time, typically from 30 minutes to 2 hours and at ambient temperature. Further prolongation of stirring has essentially no improving effect (only a decent growth of the originally formed crystals has been sometimes observed).

In the third technological step, the formed crystalline fingolimod hydrochloride is separated from the mother liquor, e.g. by filtration or centrifugation, is washed, advantageously by the antisolvent, and dried until volatile residues, if any, are removed. Further treatment, e.g. milling, is essentially not necessary, but not excluded.

The produced crystalline fingolimod hydrochloride may be used as a pharmaceutically active compound for making pharmaceutical compositions for treatment various diseases, as known in the art.

The present invention is illustrated by following non-limiting examples .

### Comparative example I (example 29 of EP 0627406 B1)

1.0 g of fingolimod hydrochloride was dissolved in
3 ml of ethanol at reflux. The solution was allowed to cool to room temperature and stirred at room temperature for about 1 hour. As a result, a solid was formed (thick cake). The solid was isolated by filtration over a P3-glass filter (reduced pressure) and air-dried overnight at R.T. An off-white, greasy to sticky solid with lumps was obtained. The yield was 0.70 g (70%).
DSC: solid-solid transitions below 75 °C and melting at 107-110 °C.

### Comparative example 2 (example 5 of WO 2000/27798)

1.0 g of fingolimod hydrochloride was dissolved in
4 ml of ethanol/ethyl acetate (1:1 V/V) at reflux. The solution was stirred and allowed to cool to room temperature. As a result, a solid was formed (thick cake, sudden crystallisation). The solid was isolated by filtration over a P3-glass filter (reduced pressure) and air-dried overnight at R.T. An off-white, greasy to sticky solid with lumps was obtained. The yield was 0.86 g (86%).
DSC: Similar to the DSC scan of Comparative example 1

### Example 1 Precipitation of fingolimod hydrochloride crystals

10 g of fingolimod hydrochloride was dissolved in
100 ml of 2-propanol at reflux (stirring). The hot solution was slowly added to
400 ml of n-heptane, mechanically stirred at 0 °C (250 rpm). As a result, a milky suspension was formed. The temperature of the mixture temporarily rose to 21 °C. After cooling, this suspension was stirred below 5 °C for few minutes.

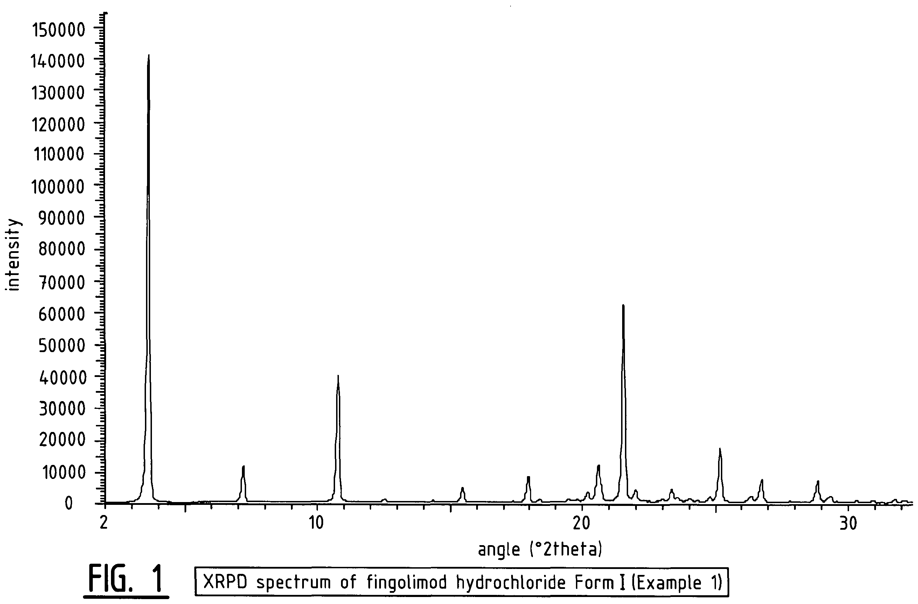

A part of the suspension (50-75 ml) was filtered through a P3-glass filter (reduced pressure, no filter clogging). The solid was air-dried overnight at room temperature. An off-white, fine powder was obtained. The flowability was clearly better compared to that of Comparative examples. The yield was 1.43 g. XRPD showed the peaks of Form I, (see Figure 1).

The remaining suspension was stirred at room temperature for an additional 1.5 hour (250 rpm). Again, a part of the suspension (50-75 ml) was filtered through a P3-glass filter (reduced pressure, no filter clogging). The solid was air-dried overnight at R.T. An off-white, fine powder with small lumps was obtained. The flowability was similar to that of the first crop. The yield was 1.47 g. XRPD showed the peaks of Form I.

The remaining suspension was stirred at room temperature for an additional 1.5 hour (250 rpm, total stirring time = 3 hr). The suspension was filtered through a P3-glass filter (reduced pressure, no filter clogging). The solid was air-dried overnight at R.T. An off-white, fine powder with small lumps was obtained. The flowability was similar to that of the preceded crop. The yield was 5.35 g. XRPD showed the peaks of Form I.

### Example 2 Precipitation of fingolimod hydrochloride crystals

0.25 g of fingolimod hydrochloride was dissolved in
10 ml of ethanol at R.T. The clear solution was dropwise added to
100 ml of di-isopropyl ether (IPE), stirred at room temperature. As a result, a solid was formed. The solid was isolated by filtration over a P3-glass filter (reduced pressure) and air-dried overweekend at R.T. A white to off-white, fine solid with lumps was obtained. The yield was 0.21 g. XRPD showed the peaks of Form I.

### Example 3 Fingolimod hydrochloride Form II and its conversion to Form I

### Approximately

0.1 g of fingolimod hydrochloride was transferred into a glass bottle. The sample was kept in an oven at 60 °C (ambient pressure) for about 2 hours. The XRPD of the solid showed peaks of the Form II.

The solid was crash cooled with a CO₂-ice/acetone bath. XRPD showed only peaks of form I.

The same experiment was repeated, but after annealing the sample was air-cooled to R.T. XRPD spectra showed peaks of both Form I and Form II. The sample was left at air and under ambient conditions and XRPD spectra were recorded after 1, 3, 14, 25 and 50 days. Only very slow and partial transition of form II into form I has been observed.

However, when the sample was subsequently kept overnight at -20 °C and warmed up to R.T., full transition of Form II into form I occurred.

## Claims

1. A process of making crystalline fingolimod hydrochloride of formula (1a), 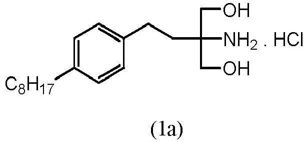 comprising a step of contacting a solution of fingolimod hydrochloride in a solvent with an antisolvent, wherein the antisolvent is a C5-C10 aliphatic, cyclic or aromatic hydrocarbon, or C4-C10 aliphatic ether, whereby crystalline fingolimod hydrochloride precipitates, and isolating crystalline fingolimod hydrochloride from the formed liquid medium.

2. The process according to claim 1, wherein the solvent is C1-C6 aliphatic alcohol.

3. The process according to claims 1-2, wherein the solvent is selected from the group comprising methanol, ethanol, n-propanol, isopropanol, and/or n-butanol, and mixtures thereof with water.

4. The process according to claims 1-3, wherein the temperature of the solution is within the range from 15 °C to the reflux temperature.

5. The process according to claims 1-4, wherein the concentration of fingolimod hydrochloride in the solvent is higher than 2.5 g/ 100 ml, preferably higher than 10g/ 100 ml.

6. The process according to claims 1-5, wherein the C5-C10 aliphatic, cyclic or aromatic hydrocarbon antisolvent is hexane, heptane, cyclohexane, benzene or toluene

7. The process according to claims 1-5, wherein the C4-C10 aliphatic ether antisolvent is diethyl ether or di-isopropyl ether

8. The process according to claims 1-7, wherein contacting the solution with the antisolvent is done by pouring the solution into stirred antisolvent.

9. The process according to claims 1-8, wherein the antisolvent is seeded with crystals of fingolimod hydrochloride Form I.

10. The process according to claims 1-9, wherein the temperature of the antisolvent is in the range from 0 to 40°C.

11. The process according to claims 1-10, wherein the ratio between the solvent and antisolvent is from about 1:2 to 1 : 20 (v/v), preferably from 1 : 4 to 1 : 10 (v/v).

12. The process according to claims 1-11 wherein the formed suspension in the solvent/antisolvent mixture is stirred after completing the mixing process for 30 minutes to 2 hours and, preferably, at ambient temperature.

## Patentansprüche

1. Verfahren zum Herstellen von kristallinem Fingolimod-hydrochlorid der Formel (1a), 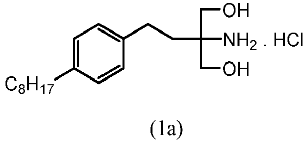 umfassend einen Schritt des Inkontaktbringens einer Lösung von Fingolimod-hydrochlorid in einem Solvens mit einem Antisolvens, wobei das Antisolvens ein C5-C10-aliphatischer, cyclischer oder aromatischer Kohlenwasserstoff oder C4-C10-aliphatischer Ether ist, wobei kristallines Fingolimod-hydrochlorid ausfällt, und Isolieren von kristallinem Fingolimod-hydrochlorid aus dem gebildeten flüssigen Medium.

2. Verfahren nach Anspruch 1, wobei das Solvens C1-C6-aliphatischer Alkohol ist.

3. Verfahren nach Ansprüchen 1-2, wobei das Solvens aus der Gruppe umfassend Methanol, Ethanol, n-Propanol, Isopropanol und/oder n-Butanol, und Gemische davon mit Wasser ausgewählt ist.

4. Verfahren nach Ansprüchen 1-3, wobei sich die Temperatur der Lösung innerhalb des Bereichs von 15°C bis zur Rückflusste mperatur befindet.

5. Verfahren nach Ansprüchen 1-4, wobei die Konzentration von Fingolimod-hydrochlorid im Solvens höher als 2,5 g/100 ml, bevorzugt höher als 10 g/100 ml ist.

6. Verfahren nach Ansprüchen 1-5, wobei das C5-C10-aliphatische, cyclische oder aromatische Kohlenwasserstoff-Antisolvens Hexan, Heptan, Cyclohexan, Benzol oder Toluol ist.

7. Verfahren nach Ansprüchen 1-5, wobei das C4-C10-aliphatische Ether-Antisolvens Diethylether oder Diisopropylether ist.

8. Verfahren nach Ansprüchen 1-7, wobei Inkontaktbringen der Lösung mit dem Antisolvens durch Schütten der Lösung in gerührtes Antisolvens durchgeführt wird.

9. Verfahren nach Ansprüchen 1-8, wobei das Antisolvens mit Kristallen von Fingolimod-hydrochlorid Form I angeimpft wird.

10. Verfahren nach Ansprüchen 1-9, wobei sich die Temperatur des Antisolvens im Bereich von 0 bis 40°C befindet.

11. Verfahren nach Ansprüchen 1-10, wobei das Verhältnis zwischen Solvens und Antisolvens von etwa 1:2 bis 1:20 (v/v), bevorzugt von etwa 1:4 bis 1:10 (v/v) beträgt.

12. Verfahren nach Ansprüchen 1-11, wobei die gebildete Suspension im Solvens/Antisolvensgemisch nach Vollenden des Mischverfahrens für 30 Minuten bis 2 Stunden, und bevorzugt bei Umgebungstemperatur gerührt wird.

## Revendications

1. Un procédé de fabrication de chlorhydrate de fingolimod cristallin de formule (la) 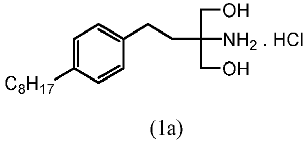 comprenant une étape de mise en contact d'une solution de chlorhydrate de fingolimod dans un solvant avec un agent antisolvant, cet agent antisolvant étant un hydrocarbure cyclique, aromatique ou aliphatique en C₅-C₁₀ ou un éther aliphatique en C₄-C₁₀, procédé dans lequel le chlorhydrate de fingolimod cristallin précipite et dans lequel le chlorhydrate de fingolimod cristallin est séparé du milieu liquide formé.

2. Le procédé selon la revendication 1, dans lequel le solvant est un alcool aliphatique en C₁-C₆.

3. Le procédé selon les revendications 1 à 2, dans lequel le solvant est choisi dans le groupe comprenant le méthanol, l'éthanol, le n-propanol, l'isopropanol et/ou le n-butanol, etleurs mélanges avec de l'eau.

4. Le procédé selon les revendications 1 à 3, dans lequel la température de la solution est comprise entre 15 °C et la température de reflux.

5. Le procédé selon les revendications 1 à 4, dans lequel la concentration dans le solvant du chlorhydrate de fingolimod est supérieure à 2,5 g/100 ml, de préférence supérieure à 10 g/100 ml.

6. Le procédé selon les revendications 1 à 5, dans lequel l'hydrocarbure cyclique, aromatique ou aliphatique en C₅-C₁₀ formant l'agent antisolvant est l'hexane, l'heptane, le cyclohexane, le benzène ou le toluène

7. Le procédé selon les revendications 1 à 5, dans lequel l'éther aliphatique en C₄-C₁₀ formant l'agent antisolvant est de l'éther diéthylique ou de l'éther diisopropylique.

8. Le procédé selon les revendications 1 à 7, dans lequel la mise en contact de la solution avec l'agent antisolvant est réalisée en versant la solution dans l'agent antisolvant sous agitation.

9. Le procédé selon les revendications 1 à 8, dans lequel l'agent antisolvant est ensemencé de cristaux de chlorhydrate de fingolimod Form I.

10. Le procédé selon les revendications 1 à 9, dans lequel la température de l'agent antisolvant est comprise entre 0 et 40°C.

11. Le procédé selon les revendications 1 à 10, dans lequel le rapport solvant/agent antisolvant est compris entre environ 1 : 2 et 1 : 20 (v/v), de préférence entre environ 1 :4 et 1 : 10 (v/v).

12. Le procédé selon les revendications 1 à 11 dans lequel la suspension formée dans le mélange solvant/agent antisolvant est agitée après la fin du procédé de mélange pendant 30 minutes à 2 heures et, de préférence, à température ambiante.
